# EUROPÄISCHE PATENTSCHRIFT

(11) **EP 1 398 315 B1**
(45) Veröffentlichungstag und Bekanntmachung des Hinweises auf die Patenterteilung: **08.08.2007**
(21) Anmeldenummer: 03022605.4
(22) Anmeldetag: 23.11.1999
(51) Int. Cl.: C07D 487/04, A61K 31/40

(54) **Antiinflammatorische Oxo- und Hydroxyderivative von Pyrrolizinen und deren Anwendung in der Pharmazie**
Anti-inflammatory oxo-derivatives and hydroxy derivatives of pyrrolizines, and their pharmaceutical use
Derivés oxo et hydroxy anti-inflammatoires de Pyrrolizines et leur application pharmaceutique

(43) Veröffentlichungstag der Anmeldung: 17.03.2004
(62) Teilanmeldung aus: 99960993.6
(73) Patentinhaber: MERCKLE GMBH, D-89079 Ulm (DE)
(72) Erfinder: Laufer, Stefan, 72070 Tübingen (DE); Tollmann, Karola, 65611 Brechen (DE); Striegel, Hans-Günter, 89134 Blaustein (DE)
(74) Vertreter: Kinzebach, Werner

(56) Entgegenhaltungen:
- CHIN B ET AL: "ML-3000 MERCKLE GMBH" CURRENT OPINION IN ANTI-FLAMMATORY AND IMMUNOMODULATORY INVESTIGATIONAL DRUGS, CURRENT DRUGS, LONDON, GB, Bd. 1, Nr. 2, 1999, Seiten 118-122, XP008008720 ISSN: 1464-8474
- LAUFER S A ET AL: "(6,7-DIARYLDIHYDROPYRROLIZIN-5-YL)ACETIC ACIDS, A NOVEL CLASS OF POTENT DUAL INHIBITORS OF BOTH CYCLOOXYGENASE AND 5-LIPOXYGENASE" JOURNAL OF MEDICINAL CHEMISTRY, AMERICAN CHEMICAL SOCIETY. WASHINGTON, US, Bd. 37, 1994, Seiten 1894-1897, XP000986196 ISSN: 0022-2623
- DANNHARDT G ET AL: "ANTIPHLOGISTISCHE 2,3-DIHYDRO-1H-PYRROLIZINE, 13. MITT STELLUNGSISOMERE DIARYLDIHYDROPYRROLIZINYL-ESSIGSAEUREN UND -HYDROXYETHYL-DERIVATE ANTIINFLAMMATORY 2,3-DIHYDRO-1H-PYRROLIZINES, XIII: ISOMERIC (DIARYL-DIHYDROPYRROLIZINYL)ACETIC ACIDS AND 2-(DIARYLDIHYDROPYRROLIZINYL)-ETHANOLS" ARCHIV DER PHARMAZIE, VCH VERLAGSGESELLSCHAFT MBH, WEINHEIM, DE, Bd. 321, 1988, Seiten 159-162, XP000994858 ISSN: 0365-6233
- DANNHARDT G ET AL: "NONSTEROIDAL ANTIINFLAMMATORY AGENTS XVIII: C-5 FUNCTIONALIZED 6,7-DIPHENYL-2,3-DIHYDRO-1H-PYRROLIZINES AS INHIBITORS OF BOVINE CYCLOOXYGENASE AND 5-LIPOXYGENASE NICHTSTEROIDALE ANTIPHLOGISTIKA 18.MITT: C-5-FUNKTIONALISIERTE 6,7-DIARYL-2,3-DIHYDRO-1H-PYRROLIZINE ALS INHIBITOREN DER RINDERCYCLOOXYGEN" ARCHIV DER PHARMAZIE, VCH VERLAGSGESELLSCHAFT MBH, WEINHEIM, DE, Bd. 327, Nr. 8, August 1994 (1994-08), Seiten 509-514, XP000990448 ISSN: 0365-6233

## Beschreibung

Die vorliegende Erfindung betrifft antiinflammatorische Hydroxyderivate von Pyrrolizinen, pharmazeutische Mittel, welche diese Verbindungen enthalten und ihre Anwendung in der Pharmazie.

Pharmakologisch wirksame Pyrrolizinverbindungen, welche die 5-Lipoxygenase (5-LO) und die Cyclooxygenase-1 und -2 (Cox-1 und Cox-2) inhibieren, sind bereits bekannt. Beispielsweise werden antiphlogistisch wirksame Pyrrolizinverbindungen beschrieben in Arch. Pharm. 319, 231-234 (1986); 318, 661-663 (1985); 318, 663-664 (1985); 319, 500-505 (1986); 319, 749-755 (1986); 321,159-162 (1988); 327, 509-514 (1994); 330, 307-312 (1997) sowie in J. Med. Chem. 1987, 30, 820-823 und 1994, 37, 1894-1897. Eine Verbindung dieses Typs ist die 6-(4-Chlorphenyl)-7-phenyl-2,3-dihydropyrrolo-[1,2-a]pyrrolverbindung ML 3000, s. Drugs of the Future, 1995, 20 (10): 1007-1009. Sie unterdrückt die Freisetzung von Leukotrienen, Thromboxanen und Prostaglandinen. Die inhibitorische Wirkung auf die Bildung der Leukotriene und der Prostaglandine ist bei dieser Struktur ausgewogen, schädliche Effekte einer reinen Hemmwirkung auf Cyclooxygenase-1 und -2 (Cox-1 bzw. Cox-2) mit vermehrter Bildung von Leukotrienen werden hier nicht beobachtet. Bei allen diesen Verbindungen ist die 1-Stellung des Pyrrolizingerüstes unsubstituiert.

Weitere Pyrrolizinverbindungen sind in der US 5,260,451 sowie in WO 95/32970; WO 95/32971; und WO 95/32972 beschrieben. Diese Verbindungen besitzen die Strukturformel

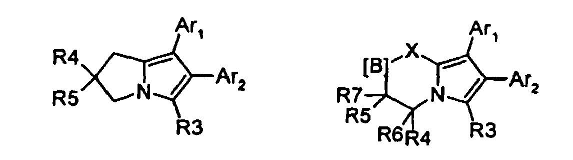

Allen Verbindungen ist ein anelliertes Diarylpyrrol-Strukturelement und ein dritter acider Rest R3 gemeinsam. Die Verbindungen zeichnen sich durch hohe Lipophilie, gute Bioverfügbarkeit und mittlere Halbwertzeiten aus.

Gemäß der allgemeinen Offenbarung in WO 95/32970 und WO 95/32971 kann X eine Carbonylgruppe bedeuten. Verbindungen dieses Typs sind jedoch nach den in den oben genannten WO-Publikationen und in der US 5,260,451 beschriebenen Verfahren nicht herstellbar.

Die US 3,705,905 offenbart Verbindungen der Formel 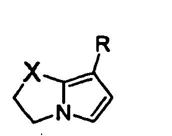 worin X für Methylen, α- oder β-Hydroxyethylen oder Carbonyl steht und R eine Hydroxymethylgruppe oder eine Formylgruppe bedeutet. Diese Verbindungen besitzen Antivirus-, Antitumor- oder immunosuppressive Aktivität.

Weitere Pyrrolizinverbindungen, die zur Behandlung thrombotischer Erkrankungen brauchbar sind, sind in US 4,546,100 und US 4, 684, 658 offenbart.

Aufgabe der vorliegenden Erfindung war es, Verbindungen zur Verfügung zu stellen, die im Vergleich zum Stand der Technik eine ebenso ausgewogene Wirkung auf hohem Niveau auf die beiden Schlüssel-Enzymsysteme der Arachidonsäurekaskade, nämlich die 5-Lipoxygenase und die Cyclooxygenasen ausüben.

Überraschenderweise wurde nun gefunden, dass diese Aufgabe durch Pyrrolizinverbindungen gelöst wird, welche in verschiedenen Positionen des Ringskelettes als polaritätserhöhende Gruppe eine Hydroxygruppe oder eine Methoxygruppe, aufweisen.

Gegenstand der vorliegenden Erfindung sind daher 2-[2-(4-Chlorphenyl)-6-hydroxymethyl-6-methyl-1-phenyl-6, 7 -dihydro-5H-pyrrolizin-3yl]-essigsäure, und die optischen Isomere, physiologisch verträglichen Salze und physiologisch leicht hydrolysierbaren Ester davon;
2-[2-(4-Chlorphenyl)-1-(4-hydroxyphenyl-6,6-dimethyl-6,7-dihydro-5H-pyrrolizin-3yl]-essigsäure, und die optischen Isomere, physiologisch verträglichen Salze und physiologisch leicht hydrolysierbaren Ester davon;
2-[2-(4-Chlorphenyl)-1-(4-methoxyphenyl)-6,6-dimethyl-6,7-dihydro-5H-pyrrolizin-3yl]-essigsäure, und die optischen Isomere, physiologisch verträglichen Salze und physiologisch leicht hydrolysierbaren Ester davon.

Die physiologisch verträglichen Salze können im vorliegenden Fall Säureadditions- oder Basenadditionssalze sein. Für Säureadditionssalze verwendet man anorganische Säuren, wie Salzsäure, Schwefelsäure oder Phosphorsäure, oder organische Säuren, wie Weinsäure, Milchsäure, Citronensäure, Äpfelsäure, Mandelsäure, Ascorbinsäure, Maleinsäure, Fumarsäure, Gluconsäure und dergleichen.

Zu Basenadditionssalzen zählen Salze der Verbindungen der Formel I mit anorganischen Basen, wie Natrium- oder Kaliumhydroxid oder mit organischen Basen, wie Mono-, Di- oder Triethanolamin.

Physiologisch leicht hydrolysierbare Ester der Verbindungen der Formel I sind beispielsweise Alkyl-, Pivaloyloxymethyl-, Acetoxymethyl-, Phthalidyl-, Indanyl- und Methoxymethylester.

Soweit die erfindungsgemäßen Verbindungen Asymmetriezentren aufweisen, sind Racemate sowie optische Isomere (Enantiomere, Diastereomere) umfasst.

Die erfindungsgemäßen Verbindungen zeigen in vitro und in vivo Hemmung der Freisetzung verschiedener Mediatoren der Arachidonsäurekaskade, insbesondere der 5-Lipoxygenase und der Cyclooxygenase-1 und -2 und daher eine ausgeprägte antiinflammatorische Aktivität. Sie eignen sich somit zur Behandlung von Erkrankungen, bei denen erhöhte Freisetzungsraten der Eicosanoid-Mediatoren für die Entstehung bzw. den progredienten Verlauf dieser Erkrankungen verantwortlich sind. Insbesondere sind die erfindungsgemäßen Verbindungen zur Behandlung von Erkrankungen des rheumatischen Formenkreises und zur Prävention von allergisch induzierten Erkrankungen brauchbar. Die erfindungsgemäßen Verbindungen stellen somit wirksame Antiphlogistika, Analgetika, Antipyretika, Antiallergika und Broncholytika dar und daher zur Prophylaxe des anaphylaktischen und septischen Schocks sowie zur Behandlung dermatologischer Erkrankungen, wie Psoriasis, Urtikaria, akute und chronische Exantheme allergischer und nicht-allergischer Genese brauchbar.

Die erfindungsgemäßen Verbindungen besitzen erhöhte chemische Stabilität, parenterale Anwendbarkeit, verbesserte enterale Bioverfügbarkeit und kürzere Halbwertszeiten.

Die erfindungsgemäßen Verbindungen können entweder als einzelne therapeutische Wirkstoffe oder als Mischungen mit anderen therapeutischen Wirkstoffen verabreicht werden. Sie können als solche verabreicht werden, im Allgemeinen werden sie jedoch in Form pharmazeutischer Mittel verabreicht, d. h. als Mischungen der Wirkstoffe mit geeigneten pharmazeutischen Trägern oder Verdünnungsmitteln. Die Verbindungen oder Mittel können oral oder parenteral verabreicht werden, vorzugsweise werden sie jedoch in oralen Dosierungsformen gegeben.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienzien enthalten, wie Bindemittel (z. B. Sirup, Akazia, Gelatine, Sorbit, Tragant oder Polyvinylpyrrolidon), Füllstoffe (z. B. Lactose, Zucker, Maisstärke, Calciumphosphat, Sorbit oder Glycin), Gleitmittel (z. B. Magnesiumstearat, Talcum, Polyethylenglykol oder Siliciumdioxid), disintegrierende Mittel (z. B. Stärke) oder Netzmittel (z. B. Natriumlaurylsulfat). Orale flüssige Präparate können in Form wässriger oder öliger Suspensionen, Lösungen, Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren, enthalten. Für die parenterale Verabreichung kann man Lösungen oder Suspensionen mit üblichen pharmazeutischen Trägern einsetzen.

Die erfindungsgemäßen Verbindungen oder Mittel können an ein Säugetier (Mensch und Tier) in Dosen von etwa 0,5 mg bis etwa 100 mg pro kg Körpergewicht pro Tag verabreicht werden. Sie können in einer Einzeldosis oder in mehreren Dosen verabreicht werden.

Das Wirkungsspektrum der Verbindungen wurde anhand folgender Testsysteme untersucht:

### Testsystem zur Bestimmung der Hemmung der 5-Lipoxygenase

Als Quelle für die 5-Lipoxygenase dienen menschliche Granulozyten. Durch Stimulation mit Calcium-Ionophor A 23187 wird LTB₄ (Leukotrien B₄) aus endogener Arachidonsäure gebildet. Die Isolierung der Granulozyten und die Durchführung der Enzymreaktion erfolgt nach bekannten Verfahren (siehe Arch. Pharm. Med. Chem. 330, 307-312 (1997)).

Das mit Heparin vor Gerinnung geschützte Blut wird über einem diskontinuierlichen Percoll^{®}-Gradienten zentrifugiert und die Granulozytenschicht abpipettiert. Nach Lyse der Erythrozyten werden die Granulozyten mehrmals gewaschen und anschließend auf eine bestimmte Zellzahl eingestellt. Die Enzymreaktion wird dann in An- bzw. Abwesenheit der Testsubstanz nach Zugabe von Ca²⁺ mit Calcium-Ionophor A 23187 gestartet. Die Synthese der Leukotriene wird nach 1,5 Minuten gestoppt. Die Proben werden abzentrifugiert, der Überstand verdünnt. Die quantitative Bestimmung von LTB₄ erfolgt mittels ELISA.

### Testsystem zur Bestimmung der Hemmung der Cyclooxygenase-1

Bei diesem Testsystem wird die von menschlichen Thrombozyten nach Zusatz von Calcium-Ionophor gebildete Prostaglandin E₂-Menge mittels ELISA bestimmt. Dabei werden die Thrombozyten nach Zentrifugation über einen diskontinuierlichen Percoll^{®}-Gradienten gewonnen. Die Enzymreaktion und die Bestimmung der gebildeten Metaboliten erfolgt prinzipiell wie bei der Bestimmung der 5-Lipoxygenase-Hemmung. Unterschiede bestehen hinsichtlich der Inkubationszeit. Weiterhin ist die Zugabe eines Thromboxansynthase-Hemmstoffes notwendig (siehe Arch. Pharm. Med. Chem. 330, 307-312 (1997)).

### Testsystem zur Bestimmung der Hemmung der Cyclooxygenase-2

COX₂ (aus Placenta des Schafs) wird mit Testsubstanz 10 min bei 4 °C vorinkubiert, dann mit Arachidonsäure (5 µM) bei 25 °C 10 min stimuliert. Als Referenz dient Diclofenac (IC₅₀ (COX₂) = 3,0 10⁻⁶ M). Die Bestimmung erfolgt in 3 Verdünnungen (10⁻⁷, 10⁻⁶, 10⁻⁵ M), Die PGE₂-Konzentrationen werden mittels ELISA quantifiziert (siehe Mitchell, J.A. et al. Proc. Nat. Acad. Sci 90: 11693-11697 (1993)).

### Bestimmung des Verteilungskoeffizienten

Der Verteilungskoeffizient P der Verbindungen wurde im System n-Octanol/Wasser nach der OECD-Guidline for Testing Chemicals, Nr. 117, bestimmt.

Die nachfolgenden Beispiele erläutern die Erfindung, ohne sie zu begrenzen.

In den Beispielen werden folgende Abkürzungen verwendet:

| | |
|---|---|
| THF | Tetrahydrofuran |
| MeOH | Methanol |
| NBS | N-Bromsuccinimid |
| DMSO | Dimethylsulfoxid |
| DMF | Dimethylformamid |
| AIBN | Azobisisobutyronitril |
| Smp | Schmelzpunkt |

### Beispiel 1

### 2-[2-(4-Chlorphenyl)-6-hydroxymethyl-6-methyl-1-phenyl-6,7-dihydro-5H-pyrrolizin-3-yl]-essigsäure

### a) 5-Hydroxymethyl-2,2,5-trimethyl-1,3-dioxan

2,2-Bis-hydroxymethyl-1-propanol (12.0 g , 0.1 Mol) wird in Toluol abs. (50 mL) suspendiert. Die auf 75 °C temperierte Suspension wird mit 2,2-Dimethoxypropan (10.9 g, 13 mL, 0.105 Mol) und Toluolsulfonsäure-Monohydrat (0.95 g , 5 mMol) versetzt. Unter intensivem Rühren wird die Mischung 5 h bei 80-85 °C gehalten. Über eine aufgesetzte Brücke wird das freiwerdende Methanol abdestilliert, anschließend wird abgekühlt.
Mit halb-gesättigter Na₂CO₃-Lsg wird die Toluol-Phase ausgeschüttelt und ausgeschiedenes Natriumtosylat mit Wasser (20 mL) wieder in Lösung gebracht. Die Phasen werden getrennt, die Wasserphase mit Toluol 2 mal (50mL) gegenextrahiert und dann verworfen. Die gesammelten Toluol-Lösungen werden mit gesättigter NaCl-Lösung gewaschen (50 mL) und über Na₂SO₄ sicc. getrocknet. Das Lösemittel wird nach Filtration vom Trockenmittel unter vermindertem Druck entfernt. Man erhält als öligen Rückstand 12.5 g Rohprodukt, von 95%iger Reinheit (nach gc Bestimmung).
Ausbeute: 75 % = 12.5 g (95 %), C₈H₁₆O₃, MW =160.21
C 59.98% H 10.07% O 29.96%
IR (NaCl) : 1/λ (cm⁻¹) = 3456, 2992, 2953, 2871, 1454, 1372, 1266, 1207, 1086, 1050, 1039, 830; -
¹HNMR (CDCl₃) : δ (ppm) = 3.65 (s; 6H, 3 CH₂), 2.64 (s, 1H, OH), 1.44 (s; 3H, CH₃), 1.40 (s, 3H, CH₃), 0.83(s;3H,CH₃),
¹³CNMR (CDCl₃): δ (ppm) = 125.3, 97.0, 65.4, 63.7, 34.3, 25.9, 21.6, 21.0, 17.7.

### b) 5-Benzyloxy-methyl-2,2,5-trimethyl-1,3-dioxan

5-Hydroxymethyl-2,2,5-trimethyl-1,3-dioxan (12.0 g, 75 mmol), gelöst in Toluol abs. (120 mL), wird mit einer Anschlämmung von NaH (60%, 4.8 g, 120 mMol) in Toluol (20 mL) versetzt, das zuvor 2 mal mit Hexan (je 10 mL) gewaschen wurde. Sobald die Wasserstoffentwicklung zum Erliegen kommt wird 20 min. auf 100°C erhitzt, anschließend in die noch heiße Reaktionslösung eine Lösung von Benzylchlorid (10.13 g, 80 mMol) in Toluol (20 mL) eingetropft und die Mischung mehrere Stunden bis zur vollständigen Reaktion refluxiert (5-10h).
Danach wird mit NaHCO₃-Lösung (120 mL) versetzt und die Phasenmischung gerührt. Die im Scheidetrichter abgetrennte Wasserphase wird 2 mal mit Toluol (160 mL) extrahiert, die vereinten Toluol-Phasen werden mit ges. NaCl-Lösung gewaschen und über Na₂CO₃ sicc. getrocknet. Die vom Trocknungsmittel filtrierte organische Phase wird im Vakuum eingeengt. Zurück bleiben 18.6 g eines gelblichen Öles (verunreinigt mit 3.7% Benzylchlorid).
Ausbeute: 99.6 % = 18.6 g (96.3 %), C₁₅H₂₂O₃, MW =250.34 C 71.97% H 8.86% O 19.17%
IR (NaCl): 1/λ (cm⁻¹)= 2990, 2860, 1453, 1370, 1208, 1089, 1028, 831, 733, 698; -
¹HNMR (CDCl₃): δ (ppm) = 7.34-7.18 (m, 5H, arom.), 4.54 (s, 2H) 3.77-3.71 / 3.58-3.52 (AB, 4H) 3.47(s, 2H) 1.42 (s, 3H) 1.38 (s, 3H) 0.90 (s, 6H).
¹³CNMR (CDCl₃) : δ (ppm) = 138.7, 129.0, 128.8, 127.3, 125.3, 97.8, 73.3, 73.1, 66.6, 34.4, 26.3, 21.4, 21.1, 18.3.

### c) 2-Benzyloxy-methyl-2-methyl-1,3-propandiol

Die Lösung von 5-Benzyloxy-methyl-2,2,5-trimethyl-1,3-dioxan (12.5 g, 50 mMol) in MeOH (250 mL) wird bei Raumtemperatur mit Trifluoressigsäure (90%, 10 mL) versetzt. Nach 1 h wird eine gc Probe auf Vollständigkeit der Spaltung überprüft, gegebenenfalls wird durch Zugabe von Trifluoressigsäure (90%) und 1-stündiges Rühren die Reaktion vervollständigt. Die Mischung wird durch Zugabe einer gesättigten Na₂CO₃ -Lösung (25 mL) neutralisiert und im Vakuum auf ein Viertel des ursprünglichen Volumens konzentriert. Nach Zugabe von Wasser bis zur Auflösung ausgeschiedener Salze (100 mL) wird im Scheidetrichter 4 mal mit Ethylacetat (400 mL) extrahiert. Die vereinten Ethylacetat-Phasen werden über Na₂SO₄ sicc. getrocknet und nach Filtrieren im Vakuum eingeengt. Man erhält 9.82 g eines hellgelben, viskosen Öls von ca. 90%iger Reinheit (gc).
Ausbeute: 93.5 % = 9.82 g (90 %), MW =210.28 C₁₂H₁₈O₃
C 68.55% H 8.63% O 22.83%
IR (NaCl): 1/λ (cm⁻¹) = 3385, 2875, 1454, 1364, 1098, 1045, 737, 698;
¹HNMR (CDCl₃) : δ (ppm) = 7.35-7.28 (5H, arom.) 4.51 (s; CH₂), 3.73-3.54 (AB, 4H, 2 CH₂), 3.46 (s; CH₂), 2.73 (s; OH), 0.82 (s; CH₃) ;
¹³CNMR (CDCl₃): δ (ppm) = 137.9, 128.5, 127.8, 127.5, 75.6, 73.6, 67.8, 40.8, 17.1.

### d) 5-Benzyloxy-methyl-5-methyl-1,3-dioxa-2-thian-2-oxid

2-Benzyloxy-methyl-2-methyl-l,3-propandiol (84.07 g, 0.4 Mol) wird in CH₂Cl₂ (420 mL) gelöst. Thionylchlorid (61.88 g, 38 mL, 0.52 Mol), gelöst in CH₂Cl₂ (100 mL) wird unter Kühlung des Ansatzes im Eiswasserbad so zugetropft, dass die HCl-Entwicklung unter Kontrolle gehalten werden kann (20 min.). Anschließend wird eine weitere Stunde bei Raumtemperatur gerührt, wobei die HCl-Entwicklung zum Erliegen kommt.
Der gesamte Ansatz wird auf Eiswasser gegossen und mit einer gesättigten Na₂CO₃-Lösung (200 mL) neutralisiert. Die alkalische Wasserphase wird noch 3 mal rasch mit CH₂Cl₂ (300 mL) extrahiert und die vereinten CH₂Cl₂-Phasen mit gesättigter Kochsalz-Lösung gewaschen, über Na₂SO₄ sicc. getrocknet, filtriert und schließlich eingeengt. Als Rückstand verbleiben 97.17 g eines leicht beweglichen, gelben Öls von 95.3% Reinheit.
Ausbeute: 94.8 % = 97.17 g (95.3 %), C₁₂H₁₆O₄S, MW =256.32
C 56.23% H 6.29% O 24.97% S 12.51%
¹HNMR (CDCl₃) : δ (ppm) = 7.37-7.24 (m; 5H, arom.), 4.60-4.54 / 3.73-3.67 (AB, 2 CH₂), 4.58 (s; CH₂), 3.63 (s; CH₂), 0.87 (s; CH₃) ; Isomer: 7.37-7.24 (m; 5H, arom.), 4.89-4.83 / 3.52-3.47 (AB, CH₂), 4.46 (s; CH₂), 3.16 (s; CH₂) 1.29 (s; CH₃) ;
¹³CNMR (CDCl₃) : δ (ppm) = 138.1, 128.3, 127.6, 127.4, 73.6, 73.3, 62.0, 35.7, 18.2
Isomer: 137.6, 128.4, 127.8, 127.6, 72.0, 71.1, 64.1, 36.0, 18.4.

### e) 4-Benzyloxy-3-hydroxymethyl-3-methyl-butyronitril

Zur Lösung von 5-Benzyloxy-methyl-5-methyl-1,3-dioxa-2-thian-2-oxid (102.53 g, 0.4 Mol) in DMSO abs. (480 mL) gibt man NaCN (25.48 g, 0.52 Mol) und erhitzt anschließend auf eine Innentemperatur von 105°C. Man rührt die sich rasch dunkel verfärbende Mischung über Nacht (16-20 h) bei dieser Temperatur und kontrolliert eine Probe mittels gc auf den Grad der Umsetzung. Im Vakuum werden aus der Reaktionsmischung 2 Drittel des eingesetzten DMSO (320 mL) abdestilliert und nach Abkühlen wird der verbleibende Rückstand auf Wasser (1.5 L) gegossen. Die alkalische Mischung wird mit verd. HCl (3%) schwach sauer gestellt (pH 4) und 4 mal mit Diethylether (1.2 L) extrahiert. Die gesammelten Ether-Phasen werden vereinigt, mit ges. NaCl-Lösung (200 mL) gewaschen und über Na₂SO₄ sicc. getrocknet. Nach Einengen der filtrierten Ether-Phase im Vakuum verbleiben 91.16 g rotbraunes Öl von 90%iger Reinheit (gc).
Ausbeute: 95.9 % = 91.16 g (90 %), C₁₃H₁₇NO₂, MW =219.29
C 71.21% H 7.81% N 6.39% O 14.59%
IR (NaCl) : 1/λ (cm⁻¹) = 3483, 2961, 2925, 2863, 2245, 1454, 1100, 740, 699;;
¹HNMR (CDCl₃) : δ (ppm) = 7.38-7.26 (m; 5H, arom.), 4.53 (s; CH₂-Ph), 3.66-3.61 / 3.45-3.44 (AB; CH₂-OH), 3.44 (s; CH₂-O-), 2.67-2.59 / 2.50-2.42 (AB; CH₂-CN), 1.01 (s ; CH3)
¹³CNMR (CDCl₃): δ (ppm) = 137.4, 128.5, 128.0, 127.6, 118.1, 75.5, 73.7, 68.6, 38.8, 23.0, 19.1.

### f) 4-Benzyloxy-3-chloromethyl-3-methyl-butyronitril

Unter Argon werden 4-Benzyloxy-3-hydroxymethyl-3-methyl-butyronitril (7.13 g, 32 mMol) und Pyridin (2.45 g, 2.5 mL, 31 mMol) in Dichlorethan abs. (15 mL) gelöst. Eine Lösung von Thionylchlorid (4.64 g, 2,8 mL, 39 mMol) in Dichlorethan abs. (6 mL) wird bei Raumtemperatur beginnend langsam zugetropft. Die Mischung erhitzt sich dabei auf 60 °C, durch Erwärmen wird die Reaktionsmischung über Nacht (16-20 h) bei dieser Temperatur gehalten. Danach wird die Mischung auf Wasser (200 mL) gegossen, dem zur Neutralisation gesättigte Na₂CO₃ -Lösung (35 mL) beigemengt wurde.
Mit 3 Portionen Ether (300 mL) wird extrahiert, die erhaltene Ether-Lösung mit ges. NaCl-Lösung (100 mL) gewaschen, über Na₂SO₄ sicc. getrocknet und eingeengt.
Die als Rückstand erhaltene schwarze Rohware (7.51 g, 91 %) von 72%iger Reinheit (gc) wird fraktionierend destilliert.
Bei 200 °C/ 2 10⁻³ mbar destillieren 6.57 g (85.5 %) hellgelbes Öl über (75%ig nach gc).

Ausbeute: 85.5 % = 6.57 g (75 %), C₁₃H₁₆ClNO, MW =237.73
C 65.68% H 6.78% Cl 14.91% N 5.89% O 6.73%
IR (NaCl): 1/λ (cm⁻¹) = 2863, 2245, 1737, 1454, 1207, 1101, 740, 699;
¹HNMR (CDCl₃) : δ (ppm) = 7.35-7.24 (m; 5H, arom.), 4.53 (s; CH₂-Ph), 3.65-3.50 (AB; CH₂-Cl) , 3.39 (CH₂-O), 2.51 (CH₂-CN), 1.17 (CH₃);
¹³CNMR (CDCl₃): δ (ppm) = 137.6, 128.4, 127.8, 127.5, 73.5, 73.1, 49.8, 39.4, 24.0, 20.0

### g) 5-Benzyl-3-(benzyloxy-methyl)-3-methyl-3,4-dihydro-2H-pyrrol

Eine Benzylmagnesiumchlorid-Lösung in Ether (224 mL 1 m, 0,224 Mol) wird auf 30 °C erwärmt und eine Lösung von 4-Benzyloxy-3-chloromethyl-3-methyl-butyronitril (75%, 40 g, 0.126 Mol) in Diethylether (140 mL) langsam zugetropft (30 min.). Die sich bildende Suspension wird nach vollständiger Zugabe durch Erwärmen am Rückfluss gehalten. Nach 2 h wird der Ether (310 mL) abdestilliert (45 min.) und anschließend durch Toluol abs. (210 mL) ersetzt. Die gelbe Toluol-Suspension wird auf 100 °C erwärmt und anschließend 2 h bei dieser Temperatur gerührt. Der auf Raumtemperatur abgekühlte Ansatz wird mit verd. HCl (10%ig, 100 mL) versetzt und gerührt bis sich neben der wässrigen und der Toluol-Phase eine dritte, ölige Phase absetzt. Ölphase und Wasserphase werden abgetrennt, die Toluol-Phase im Scheidetrichter 2 mal mit verd. HCl (100 mL) extrahiert. Die mit den HCl-Phasen vereinte Ölphase wird mit Toluol (50 mL) gewaschen und anschließend mit NaOH (32%) auf pH 8-9 eingestellt.
Nach 30 min. Rühren wird die sich absetzende ölige Basenfraktion mit Diethylether aufgenommen, die alkalische Wasserphase wird noch 3 mal mit Ether (450 mL) extrahiert. Die gesammelten Ether-Extrakte werden über Na₂SO₄ sicc. getrocknet und eingeengt. Es verbleibt ein rotbrauner öliger Rückstand von 39.64 g (96 % d. Th., nach gc 78 %)
Ausbeute: 96 % = 39.64 g (78 %), MW =293.41 C₂₀H₂₃NO
C 81.87% H 7.90% N 4.77% O 5.45%
¹HNMR (CDCl₃): δ (ppm) = 7.37-7.17 (m; arom.), 4.45 (s; CH₂) 3.74-3.63 / 3.47-3.4 (AB; CH₂), 3.63 (s; CH₂), 3.19 (s; CH₂), 2.53-2.45 / 2.17-2.09 (AB,CH₂), 1.04 (s; CH₃).
¹³CNMR (CDCl₃) : δ (ppm) = 176.7, 141.6, 138.7, 136.9, 135.7, 133.6, 130.7, 129.7, 129.2, 128.9, 128.8, 128.7, 128.6, 128.5, 127.9, 127.8, 127.7, 127.6, 127.1, 127.0, 126.9, 77.0, 73.6, 73.4, 72.7, 70.4, 65.1, 47.5, 46.6, 43.0, 42.2, 41.1, 24.0

### h) 2-(Benzyloxy-methyl)-6-(4-chloro-phenyl)-2-methyl-7-phenyl-2,3-dihydro-1H pyrrolizin

In einem 2L Kolben werden 5-Benzyl-3-(benzyloxy-methyl)-3-methyl-3,4-dihydro-2H-pyrrol (108 g, 0.368 Mol), 2-Brom-1-(4-chlorphenyl)-1-ethanon (100 g, 0.428 Mol) und NaHCO₃ (34.6 g, 0.42 Mol) in Methanol abs. (950 mL) gelöst und, auf 40 °C temperiert, insgesamt 2 d unter Lichtausschluss gerührt.
Von dem während der Reaktion gebildeten zähen, grünbraunen Bodenkörper wird die überstehende Methanol-Phase abgegossen, die Masse mit Diethylether (1L) und Wasser (800 mL) aufgenommen und verteilt. Die Wasserphase wird 2 mal mit Ether extrahiert (600 mL). Die gesammelte Ether-Lösung wird über Na₂SO₄ sicc. getrocknet und eingeengt. Lösungsmittelreste werden im Hochvakuum entfernt. Es verbleiben 124 g eines zähen sich aufblähenden Rückstands, der aus der gesuchten Verbindung besteht und der für weitere Umsetzungen ohne weitere Reinigung eingesetzt werden kann. Zur Charakterisierung wird die Substanz über SC gereinigt (Al₂O₃/Hexan-Diethylether 4:1).

Ausbeute: 78.8 % = 124 g, C₂₈H₂₆ClNO, MW =427.98
C 78.58% H 6.12% Cl 8.28% N 3.27% O 3.74% ¹HNMR (CDCl₃): δ (ppm) = 7.35-7.12 ( m; arom.) 6.67 (s; 1H), 4.54 (s; CH₂), 4.09-4.04 / 3.7-3.66 (AB; CH₂), 3.42 (s; CH₂), 3.01-2.93 / 2. 75-2.67 (AB; CH₂), 1.33 (s; CH₃);
¹³CNMR (CDCl₃): δ (ppm) = 138.2, 136.0, 135.1, 135.0, 131.0, 129.8, 129.4, 129.3, 129.1, 128.7, 128.5, 128.3, 128.2, 128.1, 127.6, 127.5, 127.4, 127.3, 125.6, 125.0, 114.2, 113.2, 76.3, 73.2, 55.6, 47.6, 30.8, 24.0,

### i) 6-(Benzyloxy-methyl)- 2-(4-chloro-phenyl-)-6-methyl-1-phenyl-6,7-dihydro-5H-pyrrolizin-3-yl-2-oxo-essigsäure-ethylester

In die Lösung von 2-(Benzyloxy-methyl)-6-(4-chloro-phenyl)-2-methyl-7-phenyl-2,3-dihydro-1H-pyrrolizin (19.02 g, 44.4 mMol) in THF abs. (240 mL) wird die Lösung von Ethyloxalylchlorid in THF (50 mL) bei Raumtemperatur zugetropft und noch 45 min. gerührt, worauf eine dc Probe (SiO₂/Diisoppropylether-Hexan 1:1) kein Edukt mehr anzeigt.
Die rosa bis lila verfärbte Lösung wird mit Wasser (240 mL) versetzt und mit Na₂CO₃-Lsg. (10%ig, 80 mL) neutralisiert. Die sich aus der gelb gefärbten Mischung abscheidende Ölphase wird im Scheidetrichter abgetrennt. Die THF-haltige Wasserphase wird 2 mal mit Diethylether (200 mL) extrahiert und die Ether-Phasen mit der Ölphase vereint. Die Ether-Lösung wird mit gesättigter NaCl-Lösung (100 mL) gewaschen, über Na₂SO₄ sicc. getrocknet und eingeengt. Als Rückstand verbleiben 21.57 g (91.9 % d. Th.) eines gelben Öls.
Ausbeute: 91.9 % = 21.57 g, C₃₂H₃₀ClNO₄, MW =528.05.
C 72.79% H 5.73% Cl 6.71% N 2.65% O 12.12%
IR (NaCl) : 1/λ (cm⁻¹) = 1746; 1637; 1450; 1427; 1250; 1093; 1063; 1014; 768; 700
¹HNMR (CDCl₃) : δ (ppm) = 7.34-6.93 (m,14H, arom.), 4.55 (s, 2H, CH₂), 4.51-4.16 (AB, CH₂), 3.62-3.55 (q, 2H, CH₂), 3.44 (s, 2H, CH₂), 3.18-2.67(AB, CH₂), 1.33 (s, 3H, CH₃), 1.09-1.02 (t, 3H, CH₃);

### j) 6-(Benzyloxy-methyl)- 2-(4-chloro-phenyl-)-6-methyl-1-phenyl-6,7-dihydro-5H-pyrrolizin-3-yl-essigsäure-ethylester

In abs. CH₂Cl₂ (300 mL) werden nacheinander 6-(Benzyloxy-methyl)- 2-(4-chloro-phenyl-)-6-methyl-1-phenyl-6,7-dihydro-5H-pyrrolizin-3-yl-2-oxo-essigsäure-ethylester (19.54 g, 37 mMol), Natriumcyanoborhydrid (17.43, 277 mMol) und Zink-II-Jodid (16.81 g, 52 mMol) eingetragen und bei Raumtemperatur gerührt. Nach 2 h zeigt eine DC Kontrolle (SiO_{2/}Diisoppropylether-Hexan 1:1) kein Edukt mehr. Daraufhin wird Wasser (300 mL) zugegeben und mit verd. Phosphorsäure (8%) auf pH4 eingestellt. Die abgetrennte Wasserphase wird noch 2 mal mit CH₂Cl₂ (200 mL) extrahiert, die vereinten CH₂Cl₂-Extrakte mit gesättigter NaCl-Lösung (100 mL) gewaschen. Nach Trocknen über Na₂SO₄ sicc. und Einengen verbleiben 19.10 g eines rotbraunen Harzes (100% d. Th.)
Ausbeute: 100 % = 19.10 g, C₃₂H₃₂ClNO₃, MW =514.07
C 74.77% H 6.27% Cl 6.90% N 2.72% O 9.34%
¹HNMR (CDCl₃): δ (ppm) = 7.34-7.02 (m,14H,3 aromat.), 4.55 (s, 2H, CH₂), 4.25-4.10 (q, 2H, CH₂), 4.07-3.68 (AB,CH₂), 3.50 (s, 2H; CH₂), 3.50-3.40 (AB,CH₂), 3.05-2.72 (AB, CH₂); 1.35 (s, 3H, CH₃), 1.30-1.12 (t, 3H, CH₃)
¹³CNMR (CDCl₃): δ (ppm) = 170.7; 138.3; 135.9; 134.6; 133.4; 131.6, 128.4; 128.2; 128.2; 128.0; 127.6; 127.5; 124.6; 123.8; 117.6; 114.6; 73.3; 61.1; 54.3; 47.5; 36.1; 31.5; 24.1; 14.2

### k) 2-(4-Chloro-phenyl-)-6-hydroxymethyl-6-methyl-1-phenyl-6,7-dihydro-5H-pyrrolizin-3-yl-essigsäure-ethylester

Die Lösung des 6-(Benzyloxy-methyl)- 2-(4-chloro-phenyl-)-6-methyl-1-phenyl-6,7-dihydro-5H-pyrrolizin-3-yl-essigsäure-ethylesters (49.23 g, 96 mMol) in abs. CH₂Cl₂ (500 mL) wird auf -35°C gekühlt und tropfenweise mit einer Lösung von Bortribromid (96 g, 383 mMol) in abs. CH₂Cl₂ (250 mL) versetzt, wobei sich die Lösung tiefrot verfärbt. Bei dieser Temperatur wird nach der vollständigen Zugabe (1 h) eine halbe Stunde gerührt und anschließend vorsichtig mit Na₂CO₃-Lösung (10%, 400 mL) bis pH 4 abgestumpft. Die CH₂Cl₂-Phase wird im Scheidetrichter abgetrennt, mit Wasser gewaschen und über Na₂SO₄ sicc. getrocknet. Nach Abdampfen des Lösemittels verbleiben 46.8 g (115 %), die in Diethylether aufgenommen werden und über eine Al₂O₂-gefüllte Säule (neutral, Aktivität II, 500 g) filtriert werden. Die Säule wird 4 mal mit einem Gemisch aus Ethylacetat und Ether (1:1, 400 mL) nachgespült. Nach Einengen der gesammelten Filtrate werden als Rückstand 37.12 g eines rot-gelben Öls erhalten (91.2 %).
Ausbeute: 91.2 % = 37.12 g, C₂₅H₂₆ClNO₃, MW =423.94
C 70.83% H 6.18% Cl 8.36% N 3.30% O 11.32% IR (KBr): 1/λ (cm⁻¹) = 3464; 2925; 1732; 1602; 1529; 1487; 1450; 1176; 1096; 1029; 1013; 831; 765; 700;
¹HNMR ([d₄]-MeOH): δ (ppm) = 7.26-6.99 (m, 9H, aromat.), 4.20-4.05 (q, 2H, CH₂), 4.00-3.65 (AB,CH₂), 3.52 und 3.54 (2 s, 4H, 2 CH₂), 3.02-2.63 (AB, CH₂), 1.29 (s, 3H, CH₃), 1.29-1.17 (t, 3H, CH₃);
¹³CNMR ([d₄]-MeOH) δ (ppm) = 172.7; 137.5; 136.6; 134.6; 132.8; 132.6; 129.3; 129.2; 129.0; 125.7; 124.7; 119.1; 116,0; 69.0; 62.2; 54.8; 36.3; 32.1; 23.5; 14.5;

### 1) 2-(4-Chloro-phenyl-)-6-hydroxymethyl-6-methyl-1-phenyl-6,7-dihydro-5H-pyrrolizin-3-yl-essigsäure

Der in Ethanol (160 mL) gelöste 2-(4-Chloro-phenyl-)-6-hydroxymethyl-6-methyl-1-phenyl-6,7-dihydro-5H-pyrrolizin-3-yl-essigsäure-ethylester (74.0 g, 175 mMol) wird bei Raumtemperatur mit verd. NaOH (10%ig, 290 mL) versetzt. Nachdem 45 min. bei Raumtemperatur gerührt wurde, wird mit verd. H₃PO₄ (8%, 150 mL) abgestumpft (pH9). Das sich abscheidende Na-Salz wird über Filtration gesammelt, trocken gesaugt und mit Diisopropylether (200 mL) wird anhaftender Ester abgewaschen. Das gereinigte Salz wird in Wasser resuspendiert (300 mL) und mit verd. H₃PO₄ (8%, 380 mL) bei pH 2-3 als Säure abgeschieden. Die gefällte Säure wird mit Wasser bis zur neutralen Reaktion der Waschflüssigkeit gewaschen und schließlich über P₂O₅ im Vakuum getrocknet (14.6 g, 19.8 %). Natriumsalz-Mutterlauge und Filtrate werden gesammelt, mit verd. H₃PO₄ (8%, 380 mL) auf pH 2-3 gebracht und die abgeschiedene Säure in Ether (400 mL) aufgenommen. Nach Waschen mit gesätt. NaCl-Lösung (100 mL) und Trocknen über Na₂SO₄ sicc. wird das Lösungsmittel im Vakuum abgedampft. Der verbleibende Rückstand (45.01 g, 53%ig) wird aus Diisopropylether (250 mL) kristallisiert. Es kristallisieren 21.36 g mit einer Reinheit von 71.9%.
Die beiden erhaltenen Feststoffe werden zusammen mit einer CH₂Cl₂-THF-Ethylacetat-Mischung (1:1:3, 200 mL) in der Wärme mit Ultraschall behandelt und nach Absaugen (23.2 g 92%ig) aus Aceton (0.7L) umkristallisiert. Man erhält letztendlich 19 g Säure (25 %) mit einer Reinheit von 96.8%.
Ausbeute: 25 % = 19 g, C₂₃H₂₂ClNO₃, MW =395.89
C 69.78% H 5.60% Cl 8.96% N 3.54% O 12.12%
IR (KBr): 1/λ (cm⁻¹) = 3269; 2958; 1683; 1603; 1531; 1485; 1396; 1305; 1290; 1103; 1034; 1014; 831; 762; 694;
¹HNMR ([d_{6]}-DMSO): δ (ppm) = 7.31-7.03 (m, 9H, aromat.), 6.01 (2 OH, br.), 4.06-3.67 (AB, CH₂), 3.54 (s, 2H, CH₂), 3.50 (s, 2H, CH₂), 3.07-2.68 (AB, CH₂), 1.31 (s, 3H, CH₃);
¹³CNMR ([d₆]-DMSO): δ (ppm) = 171.8; 135.9; 135.0; 133.2; 131.3 (CH); 130.3; 128.2 (CH); 128.1 (CH); 127.6 (CH); 124.4 (CH); 122.0; 118.6; 113.3; 67.1 (CH₂) ; 53.5 (CH₂) ; 48.0(Cq); 35.0 (CH₂); 31.0(CH₂) ; 23.5 (CH₃)

### Beispiel 2

### 2-(4-Chlorphenyl)-1-(4-hydroxyphenyl)-6,6-dimethyl-6,7-dihydro-pyrrolizin-3-yl-essigsäure

### a) 2-(4-Methoxybenzyl)-4,4-dimethyl-1-pyrrolin

Magnesiumspäne (für Grignard, 48,6 g, 2 Mol) werden unter Argon in Diethylether (480 mL) suspendiert; 4-Methoxybenzylchlorid (31.2 g, 0.2 Mol) wird in Diethylether (200 mL) gelöst und 5-10 mL davon werden zum Magnesium gegeben. Nach Zugabe eines Jodkristalles (0.05 g, 0,2 mMol) wird ohne zu Rühren erhitzt bis sich die Reaktionsmischung trübt. Die restliche 4-Methoxybenzylchlorid-Lösung wird unter Rühren rasch zugetropft und die Ansatzmischung anschließend noch 1 h unter Rückfluss erhitzt. Nach dem Abkühlen wird der etherische Überstand unter Argon-Schutzatmosphäre vom Magnesium in ein zweites Reaktionsgefäß abdekantiert und dabei über Glaswolle filtriert.
Zur so erhaltenen Grignardlösung wird tropfenweise eine Lösung von 4-Chlor-3,3-dimethyl-butyronitril (13.1 g, 0.1 Mol) in Diethylether (60 mL) bei Raumtemperatur zugegeben und nach vollständiger Zugabe wird das Lösungsmittel (Diethylether) über eine DestillationsBrücke bis zu einer Sumpf-Temperatur von 45-50°C abdestilliert. Mit wasserfreiem Toluol (450 mL) wird aufgefüllt und bis zu einer Sumpf-Temperatur von 90-95°C der restliche Ether ausgetrieben. Nach 30 min. bei dieser Temp. lässt man in einem Eisbad abkühlen und zersetzt die Reaktionsmischung durch Zugabe von HCl (10%ig, 200 mL). Im Scheidetrichter wird die HCl-saure Phase abgetrennt, die Toluolphase noch 2 mal mit HCl (10 %, 150 mL) extrahiert. Die vereinigten salzsauren Extrakte werden mit Diethylether (100 mL) gewaschen und mit konz. Ammoniaklösung (25%) alkalisch gestellt (pH 9-10). Die sich aus der Wasserphase abscheidende Pyrrolinbasen-Fraktion wird in Diethylether aufgenommen (2 x 100 mL), mit Wasser (2 x 50 mL) gewaschen und über wasserfreiem Na₂CO₃ getrocknet. Nach dem Entfernen des Lösungsmittels im Vakuum verbleiben 21,7 g 2-(4-Methoxybenzyl)-4,4-dimethyl-1-pyrrolin (65 % bez. auf eingesetztes Nitril) als rötlich-braunes Öl, mit einer Reinheit von 65 % (nach gc.).
Ausbeute: 100 % = 21,7 g, C₁₄H₁₉NO, MW = 217.31
calc.: C 77.38% H 8.81% N 6.45% O 7.36%
IR (NaCl): 1/λ (cm⁻¹) = 2953, 2835, 1511, 1247, 1176, 1035,
¹HNMR (CDCl₃) : δ (ppm) = 7,15-7,11(AA',2H, arom.); 6,86-6,82(BB' ,2H, arom.) ; 3,78 (s, 3H, OCH₃) ; 3,54 (s, 4H, 2 CH₂) ; 2,20 (s, 2H, CH₂); 1, 01 (s, 6H, CH₃);

### b) 6-(4-Chlorphenyl)-2,2-dimethyl-7-(4-methoxyphenyl)-2,3-dihydro-1H-pyrrolizin

In die Lösung von 2-(4-Methoxybenzyl)-4,4-dimethyl-1-pyrrolin (21,7 g, 65%, 0,065 Mol) in MeOH (200 mL) wird 2-Brom-(4-chlorphenyl)-1-ethanon (15.2 g, 0,065 Mol) in kleinen Portionen unter Rühren eingetragen. Der klaren Lösung der Komponenten wird anschließend NaHCO₃ (6.5 g) zugefügt und über Nacht (16 h) gerührt. Dabei ausgefallenes Kristallisat wird abfiltriert (8.6 g), die Mutterlauge wird im Vakuum auf ein Viertel des Ausgangsvolumens eingeengt und nochmals zur Kristallisation kalt gestellt. Das Nachkristallisat (2 g) wird mit dem ersten vereint, insgesamt werden so 10,6 g (47%) kristallines 6-(4-Chlorphenyl)-2,2-dimethyl-7-(4-methoxyphenyl)-2,3-dihydro-1H-pyrrolizin erhalten.
Ausbeute: 47 % = 10,6 g, C₂₂H₂₂ClNO, MW = 351.88 calc.: C 75.10% H 6.30% Cl 10.08% N 3.98% O 4.55%
IR (NaCl): 1/λ (cm⁻¹): 2956-2870, 1523, 1504, 1242, 1177, 826,
¹HNMR (CDCl₃) : δ (ppm) = ppm: 7,17(s, 4H, arom.); 7,12-7,05 (AA', 2H, arom.); 6,84-6,78 (BB', 2H, arom.); 6,67 (s,1H, pyrrol); 3,79 (s, 3H, OCH₃); 3,73 (s*,* 2H, NCH₂); 2,76 (s, 2H, CH₃); 1,27 (s, 6H, CH₃) ;

### c) 2-{2-(4-Chlorphenyl)-1-(4-methoxyphenyl)-6,6-dimethyl-6,7-dihydro-5H-pyrrolizin-3-yl]-oxoessigsäure-ethylester

Zum 6- (4-Chlorphenyl) -2, 2-dimethyl-7- (4-methoxyphenyl) -2,3-dihydro-1H-pyrrolizin (10,6 g, 0.03 Mol), klar gelöst in THF (100 mL), wird im Eisbad unter Rühren eine Lösung von Ethyl-Oxalylchlorid in THF (20 mL)ge tropft und anschließend 1 h gerührt. Das ausgefallene Produkt (Kristallisat a = 9.2 g) kann abgesaugt werden. Die THF Lösung wird bis zur Trockne eingedampft und der Rückstand aus Diisopropylether umkristallisiert (Kristallisat b = 1.7 g). Zusammen werden 10.9 g (80 %) 2-(2-(4-Chlorphenyl)-1-(4-methoxyphenyl)-6,6-dimethyl-5,6-dihydro-5H-pyrrolizin-3-yl]-oxoessigsäure-ethylester als Kristallisat erhalten.
Ausbeute: 80 % = 10.9 g, C₂₆H₂₆ClNO₄, MW = 451.95 calc.: C 69.10%; H 5.80% Cl 7.84% N 3.10% O 14.16%
IR (NaCl): 1/λ (cm⁻¹) = 2954-2843, 1739, 1610, 1251, 1250, 1071, 838,
¹HNMR (CDCl₃) : δ (ppm) = 7,28-7,25 (AA', 2H, arom.); 7,17-7,14 (AA', 2H, arom.); 6,89-6,86 (BB', 2H, arom.); 6,76-6,73 (BB', 2H, arom.): 4,23 (s, 2H, CH₂N); 3,75 (s, 3H, OCH₃); 3,58 (q, 2H, OCH₂); 2,81 (s. 2H, CH₂); 1,32 (s, 6H, C(CH₃)₂); 1,06 (t, 3H, CH₂CH₃);

### d) 2-[2-(4-Chlorphenyl)-1-(4-methoxyphenyl)-6,6-dimethyl-6,7-dihydro-5H-pyrrolizin-3-yl] -essigsäure-ethylester

2-[2-(4-Chlorphenyl)-1-(4-methoxyphenyl)-6,6-dimethyl-5,6-dihydro-5H-pyrrolizin-3-yl]-oxoessigsäure-ethylester (4.61 g, 0.010 mMol) wird in abs. CH₂Cl₂ (80 mL) gelöst und anschließend wird Zink-IIiodid (4.46 g, 0.014 Mol ) eingerührt. Die zunächst rote Farbe des Ansatzes verändert sich nach braun. Nach Zugabe von NaCNBH₃ (5.14 g, 0.07 Mol) hellt sich die Farbe der bei Raumtemperatur gerührten Suspension im Verlauf der Reaktion auf über orange nach gelb (5.5 h). Nach vollständiger Umsetzung (dc SiO₂ / Isoether-n-Hexan 7:3) werden Wasser (100 mL) und verd. H₃PO₄ (8%) bis zur schwach sauren Reaktion (pH 5) zugegeben. Die CH₂Cl₂-Phase wird im Scheidetrichter abgetrennt und die Wasserphase mit Ethylacetat (3 mal 30 mL) extrahiert. Die beiden organischen Phasen werden vereint, mit ges. NaCl-Lösung (2 mal 50 mL) gewaschen, über Na₂SO₄ sicc. getrocknet und im Vakuum zur Trockne eingedampft. Der Rückstand kristallisiert aus Diisopropylether (20 mL), man erhält 3.0 g (67 %) 2-[2-(4-Chlorphenyl)-1-(4-methoxyphenyl)-6,6-dimethyl-5,6-dihydro-5H-pyrrolizin-3-yl]-essigsäure-ethylester mit Schmelzpunkt 105 °C.
Ausbeute: 67 % = 3.0 g, C₂₆H₂₈ClNO₃, MW = 437.97 Schmp. 105 °C
IR (NaCl) : 1/λ (cm⁻¹) = 2954-2833, 1725, 1531, 1505, 1292, 1178, 1033, 838; -
¹HNMR (CDCl₃) : δ (ppm) = 7,26-7,22 (AA', 2H, arom.); 7,14-7,10 (AA', 2H, arom.); 6.,99-6,95 (BB', 2H, arom.); 6,76-6,72 (BB', 2H, arom.); 4,18 (q, 2H, CH₂); 3,76 (s, 3H, OCH₃) ; 3,74 (s, 2H, CH₂); 3,5 (s, 2H, CH₂); 2,8(s, 2H, CH₂); 1,31-1,24 (s + t, 9H, C (CH₃)₂ + CH₂CH₃).

### e) 2-[2-(4-Chlorphenyl)-1-(4-hydroxyphenyl)-6,6-dimethyl-6,7-dihydro-5H-pyrrolizin-3-yl]-essigsäure-ethylester

2-[2-(4-Chlorphenyl)-1-(4-methoxyphenyl)-6,6-dimethyl-5,6-dihydro-5H-pyrrolizin-3-yl]-essigsäure-ethylester (0.9 g, 2 mMol) wird in CH₂Cl₂ (20 mL) gelöst und auf -30 °C abgekühlt. Bei dieser Temperatur wird eine Lösung von BBr₃ (0.84 mL, 8.8 mMol) in CH₂Cl₂ (15 mL) mit einer Spritze durch ein Septum in kleinen Anteilen (über 10-15 min.) zugegeben. Die Innentemperatur sollte dabei -25 °C nicht überschreiten. Bei -30 °C wird 3 h gerührt, (DC-Probe: Si0₂, n-Hexan/Ether 7:3, Produkt rf=0.2, Edukt rf=0.35) anschließend wird durch Eingießen der kalten Reaktionslösung in eine halbgesättigte NaHCO₃-Lösung die Reaktion abgebrochen. Mit Ethylacetat wird dreimal extrahiert (60 mL) und die gesammelte organische Phase mit ges. NaCl-Lösung (50 mL) gewaschen, über Na₂SO₄ sicc. getrocknet und das Lösungsmittel im Vakuum entfernt. Der zurückbleibende aufschäumende Rückstand wird aus MeOH kristallisiert : 0.54 g (63.7% d. Th.).
Ausbeute: 63.7 % = 0.54 g, C₂₅H₂₆ClNO₃, MW = 423.94
IR (NaCl): 1/λ (cm⁻¹) = 3408.3 ; 3227.0 ; 2957.6 ; 2932.2 ; 2869.5 ; 2901.2 ; 1732.0 ; 1713.0 ; 1531.1 ; 1506.0 ; 1262.4 ; 1214.5 ; 1172.6 ; 829.6; -
¹HNMR (Aceton-d6): δ (ppm) = 8.1 (OH, phenol.) ; 7.30-6.64 (m, 8H); 4.15-4.11 (q, 2H, CH₂) , 3.76(s, 2H, CH₂); 3.52 (s, 2H, CH₂); 2.76 (s, 2H, CH₂) ; 1.27 (s, 6H, C(CH₃)₂), 1.06 (t, 3H, OCH₂CH₃).
¹³CNMR (Aceton -d6): δ (ppm) =171.1 ; 136.3 ; 133.6 ; 132.4 ; 131.5 ; 129.9 ; 128.7 ; 128.2 ; 123.3 ; 118.5 ; 115.6 ; 115.1 ; 61.2 ; 58.6 ; 43.7 ; 40.7 ; 31.7 ; 27.7 ; 14.4.

### f) 2-[2-(4-Chlorphenyl)-1-(4-hydroxyphenyl)-6,6-dimethyl-6,7-dihydro-5H-pyrrolizin-3-yl]-essigsäure

2-[2-(4-Chlorphenyl)-1-(4-hydroxyphenyl)-6,6-dimethyl-5,6-dihydro-5H-pyrrolizin-3-yl]-essigsäure-ethylester (0.42 g , 1 mMol) wird unter Argon in einer Mischung aus Ethanol (15 mL) und NaOH (3 mL, 10 %) gelöst und 15 min. unter Rückflusskochen hydrolysiert.
Die ethanolische Lösung wird mit Wasser (50 mL) verdünnt und mit verd HCl (10 %) sauer gestellt. Die blau gefärbte Lösung wird mit Ethylacetat (3x20 mL) extrahiert, die gesammelte organische Phase mit ges. NaCl-Lösung (50 mL) gewaschen, über Na₂SO₄ sicc. getrocknet und das Lösungsmittel im Vakuum entfernt. Der Rückstand kristallisiert aus MeOH (5 mL), 80 mg Kristalle (28,6 %) werden isoliert (DC: Si02 - Ether/Hexan 2:1).
Ausbeute: 28.6% = 0.08 g; C₂₆H₂₈ClNO₃, MW = 437.97
IR (NaCl) : 1/λ (cm⁻¹) = 3303.3, 2956.8, 2928.7, 1679.0 1687.0, 1506.1, 1265.1, 1234.2, 827.3.
¹HNMR (Aceton-d6): δ (ppm) = 8.1 (OH, 1H, phenol.); 7.28-6.65 (m, 8H, 2 AA'BB'-Systeme), 3.79 (s, 2H, CH₂), 3.54 (s, 2H, CH₂), 2.77 (s, 2H, CH₂), 1.28 (s, 6H, C(CH₃)₂).
¹³CNMR (Aceton-d6): δ (ppm) = 172.2, 136.6, 133.7, 132.6, 131.6, 130.0, 128.8, 128.7, 128.4, 123.4, 119.0, 115.7, 115.3, 58.7, 43.8, 40.8, 31.4, 27.9.

### Beispiel 3

### 2-[2-(4-Chlorphenyl)-1-(4-methoxyphenyl)-6,6-dimethyl-6,7-dihydro-5H-pyrrolizin-3-yl]-essigsäure

Zum 6-(4-Chlorphenyl)-2,2-dimethyl-7-(4-methoxyphenyl)-2,3-dihydro-1H-pyrrolizin (Beispiel 2 b; 0.71 g, 2 mMol), klar gelöst in Tetrahydrofuran (THF) (5 mL), wird im Eisbad unter Rühren eine Lösung von Oxalylchlorid (380 mg, 3 mMol) in THF (0.5 mL) getropft und anschließend 15 min. gerührt. Mit Wasser (2 mL) wird überschüssiges Oxalylchlorid zersetzt. Nach Zutropfen von Hydrazinhydrat (1.3 mL, 80 %) wird 30 min. gerührt, dann Diethylenglycol (4 mL) zugesetzt und bei einer Badtemperatur von 100 °C das enthaltene THF abdestilliert. Man kühlt auf 80 °C und gibt KOH (2.0 g, 35 mMol) zu und erhitzt die Mischung in einem auf 160 °C temperierten Ölbad. Am Wasserabscheider gehen Reste des THF, Wasser und der Überschuss an Hydrazin über, der Ansatz schäumt, entfärbt sich und Gas wird frei. Die Temperatur wird 1 h gehalten, dann wird abgekühlt und mit Wasser (20 mL) verdünnt. Auf Ansäuern mit konz HCl scheidet sich die freie Säure ab, die abgesaugt, mit Wasser gewaschen und bei 60 °C getrocknet wird.
Ausbeute: 0.65 g (80 %), C₂₄H₂₄ClNO₃; MW = 409.92.
IR (KBr): 1/λ [cm⁻¹] = 2956-2841, 1725, 1709, 1505, 1244, 1177, 1031, 827,
¹HNMR (Aceton-d6): δ (ppm) =: 7,26-7,23(d,2H,arom.); 7,14-7,10 (d,2H,arom.); 6,99-6,95 (d, 2H, arom.); 6,76-6,72(d,2H,arom.); 3,74 (s, 3H, OCH₃); 3,70 (s, 2H, CH₂) ; 3,57 (s, 2H, CH₂); 2,28 (s, 2H, CH₂) ; 1,29 (s; 6H, CH₃)

## Patentansprüche

1. 2-[2-(4-Chlorphenyl)-6-hydroxymethyl-6-methyl-1-phenyl-6,7-dihydro-5H-pyrrolizin-3yl]-essigsäure, und die optischen Isomere, physiologisch verträglichen Salze und physiologisch leicht hydrolysierbaren Ester davon.

2. 2-[2-(4-Chlorphenyl)-1-(4-hydroxyphenyl-6,6-dimethyl-6,7-dihydro-5H-pyrrolizin-3yl]-essigsäure, und die optischen Isomere, physiologisch verträglichen Salze und physiologisch leicht hydrolysierbaren Ester davon.

3. 2-[2-(4-Chlorphenyl)-1-(4-methoxyphenyl)-6,6-dimethyl-6,7-dihydro-5H-pyrrolizin-3yl]-essigsäure, und die optischen Isomere, physiologisch verträglichen Salze und physiologisch leicht hydrolysierbaren Ester davon.

4. Pharmazeutisches Mittel, enthaltend wenigstens eine Verbindung nach einem der Ansprüche 1 bis 3, gegebenenfalls zusammen mit pharmazeutisch akzeptablen Träger- und/oder Zusatzstoffen.

5. Verwendung wenigstens einer Verbindung nach einem der Ansprüche 1 bis 3 zur Herstellung eines pharmazeutischen Mittels zur Prävention von allergisch induzierten Erkrankungen oder zur Behandlung von Erkrankungen des rheumatischen Formenkreises.

## Claims

1. 2-[2-(4-Chlorophenyl)-6-hydroxymethyl-6-methyl-1-phenyl-6,7-dihydro-5H-pyrrolizin-3-yl]-acetic acid and the optical isomers, physiologically acceptable salts and physiologically readily hydrolysable esters thereof.

2. 2-[2-(4-Chlorophenyl)-1-(4-hydroxyphenyl-6,6-dimethyl-6,7-dihydro-5H-pyrrolizin-3-yl]-acetic acid and the optical isomers, physiologically acceptable salts and physiologically readily hydrolysable esters thereof.

3. 2-[2-(4-Chlorophenyl)-1-(4-methoxyphenyl-6,6-dimethyl-6,7-dihydro-5H-pyrrolizin-3-yl]-acetic acid and the optical isomers, physiologically acceptable salts and physiologically readily hydrolysable esters thereof.

4. Pharmaceutical composition, containing at least one compound according to any of Claims 1 to 3, optionally together with pharmaceutically acceptable carriers and/or additives.

5. Use of at least one compound according to any of Claims 1 to 3 in the preparation of a pharmaceutical composition for the prevention of allergically induced disorders or for the treatment of disorders of the rheumatic type.

## Revendications

1. Acide 2 [2-(4-chlorophényl) 6-hydroxyméthyl 6-méthyl 1-phényl 6,7-dihydro 5H-pyrrolizin 3-yl] acétique et ses isomères optiques ainsi que leurs sels physiologiquement compatibles et leurs esters à hydrolyse physiologique facile.

2. Acide 2 [2-(4-chlorophényl) 1-(4-hydroxyphényl) 6,6-diméthyl 6,7-dihydro 5H-pyrrolizin 3-yl] acétique et ses isomères optiques ainsi que leurs sels physiologiquement compatibles et leurs esters à hydrolyse physiologique facile.

3. Acide 2 [2-(4-chlorophényl) 1-(4-methoxyphényl) 6,6-diméthyl 6,7-dihydro 5H-pyrrolizin 3-yl] acétique et ses isomères optiques ainsi que leurs sels physiologiquement compatibles et leurs esters à hydrolyse physiologique facile.

4. Produits pharmaceutiques, comportant au moins un composé suivant l'une quelconque des revendications 1 à 3, éventuellement complété par un véhicule et/ou des additifs pharmaceutiquement compatibles.

5. Utilisation de l'un au moins des composés suivant l'une quelconque des revendications 1 à 3 pour la préparation d'un produit pharmaceutique pour la prévention de maladies induites par allergie ou pour le traitement de maladies à forme rhumatismale.
